# EUROPÄISCHE PATENTANMELDUNG

(11) **EP 3 272 385 A1**
(43) Veröffentlichungstag der Anmeldung: **24.01.2018**
(21) Anmeldenummer: 17182463.4
(22) Anmeldetag: 20.07.2017
(51) Int. Cl.: A61M 25/01, A61M 27/00, A61M 25/02, A61M 25/00

(54) **KATHETER ZUR INTERMITTIERENDEN SELBST-KATHETERISIERUNG**

(30) Priorität: 21.07.2016 DE 102016113527
(71) Anmelder: UROMED KURT DREWS KG, 22113 Oststeinbek (DE)
(72) Erfinder: LE CERF, Nils, 22609 Hamburg (DE); WITTE, Lars, 22145 Hamburg (DE)
(74) Vertreter: Meissner Bolte Partnerschaft mbB

(57) **Zusammenfassung**

Die Anmeldung betrifft einen Katheter (10) zur intermittierenden Katheterisierung, wobei der Katheter einen Kathetertubus (20) und ein Einführelement (22) umfasst, und an seinem distalen Endbereich (36) zwei oder mehr flexible, längliche Halteelemente (24) umfasst, deren proximale Enden (40) um den Katheter herum an dem Katheter angeordnet sind und die in Längsrichtung an den Penisschaft (14) und optional an die Eichel anlegbar sind. Darüber hinaus umfasst die Anmeldung ein Verfahren zur intermittierenden Katheterisierung.

## Beschreibung

### Hintergrund

Die Erfindung betrifft einen Katheter zur intermittierenden Katheterisierung, d. h. Ableitung von Urin, der im Oberbegriff des Anspruchs 1 genannten Art, insbesondere einen Katheter zur intermittierenden Selbst-Katheterisierung.

Bei Patienten mit Blasenfunktionsstörungen kann es notwendig sein, die Urinableitung regelmäßig mit Hilfe eines Katheters durchzuführen. Neurogene Blasenfunktionsstörungen treten z. B. bei Patienten mit Querschnittslähmung z. B. nach einer Rückenmarksverletzung auf. Das zur Blasenentleerung notwendige Kontrahieren der Blasenmuskulatur und der Blasenschließmuskel können bei solchen Patienten oft gar nicht mehr oder nur noch zeitweise bewusst gesteuert werden. Die Patienten sind daher auf Katheter angewiesen, mit deren Hilfe sie intermittierend, z. B. etwa alle 3 Stunden, Ihre Harnblase entleeren können (Intermittierender Selbstkatheterismus, ISK).

Um in den Alltag der Patienten komfortabel integriert werden zu können, sollten die Katheter steril sein und vorzugsweise eine möglichst geringe Keimverschleppung aus der der Harnröhre in die Harnblase sicherstellen und gleichzeitig auch von motorisch eingeschränkten Patienten einfach gehandhabt werden können.

Keime können beim Katheterisierungsvorgang während des Vorschiebens des Katheters in die Blase gelangen, insbesondere da wesentliche Teile der Keime sich beim Mann in den ersten 1,5 cm des Harnröhreneingangs befinden. Von dort können die Keime in den Rest der Harnröhre und die Harnblase gelangen. Aus diesem Grunde werden im Stand der Technik im ISK-Bereich Katheter mit Einführelementen eingesetzt, die eine Vorlaufspitze umfassen, die in diesen Anfangsbereich der Harnröhre eingeschoben werden kann und den keimbelasteten Bereich somit überbrückt. Anschließend wird der Kathetertubus durch eine flexibel schließbare Öffnung in der Vorlaufspitze in die Harnröhre und bis in die Blase eingeschoben. Auch die Gefahr eines Herausrutschens des Katheters kann durch das Einführelement reduziert werden, was Patienten mit motorischen Einschränkungen die intermittierende Selbst-Ableitung von Urin deutlich erleichtert. Die Einführelemente stellen somit eine deutliche Verbesserung der ISK Katheter dar. Beispielhafte Einführelemente sind z. B. in US 2014/0276661 A1 beschrieben.

Trotz dieser Verbesserungen fällt es gerade Patienten mit motorischen Einschränkungen, wie z. B. Verkrampfungen der Muskulatur, oft schwer, die ISK Katheter einzuführen und während des Ableitvorgangs am Penis zu halten. Es wäre daher wünschenswert, Katheter derart weiterzuentwickeln, dass eine gute Sicherheit für das Einführen und ein großer Haltekomfort auch für motorisch eingeschränkte Patienten gewährleistet werden.

US 4,419,097 beschreibt ein zylindrisches Gehäuse, das auf der Penisspitze und dem distalen Schaftende des Penis befestigt werden kann und das Verrutschen eines Katheters bei bettlägerigen Patienten verhindern soll. Das Gehäuse ist jedoch steif ausgebildet, so dass die Größe des Gehäuses an den jeweiligen Patienten angepasst werden muss. Darüber hinaus führt die Steifigkeit dazu, dass das Gehäuse ein großes Verpackungsvolumen einnimmt und somit für den alltäglichen Transport wie ISK Patienten ihn benötigen, z. B. in der Jackentasche, ungeeignet ist.

US 2011/0230851 A1 schlägt vor, selbstklebende Dichtungsschichten zu verwenden, um einen Katheter am Penis eines motorisch eingeschränkten Patienten zu fixieren. Hierzu sollen zunächst eine Vielzahl selbstklebender Blätter an die Eichel (*Glans penis*) geklebt werden und diese Blätter anschließend mit größeren selbstklebenden Siegeln an der Eichel fixiert werden. Hierdurch soll ein flüssigkeitsdichter Verschluss zwischen Penis und Katheter geschaffen werden. Gleichzeitig soll die Beweglichkeit der Vorhaut (*Praeputium*) nicht eingeschränkt werden. Da dieser Katheter nur an der *Glans penis* befestigt ist und aus einer relativ komplexen und klebenden Anordnung von Halteelementen besteht, ist das Einführen, Halten und Entfernen des Katheters für motorisch eingeschränkte Patienten jedoch mit erheblichen Schwierigkeiten verbunden.

Aufgabe der vorliegenden Erfindung ist es daher, einen gattungsgemäßen Katheter bereitzustellen, der zur intermittierenden Selbst-Ableitung von Urin durch einen motorisch eingeschränkten Mann geeignet ist und diesem eine schnelle, zuverlässige und hygienische Handhabung ermöglicht und darüber hinaus unauffällig und mit geringem Transportvolumen zu transportieren ist.

### Beschreibung der Erfindung

Gelöst wird die Aufgabe durch Katheter zur intermittierenden Katheterisierung mit den Merkmalen des Anspruchs 1.

Die Erfindung betrifft somit einen Katheter zur intermittierenden Katheterisierung, insbesondere zur intermittierenden Selbst-Katheterisierung, wobei der Katheter einen Kathetertubus und ein Einführelement umfasst, dadurch gekennzeichnet, dass der Katheter in seinem distalen Endbereich zwei oder mehr flexible, längliche Halteelemente umfasst, deren proximale Enden um den Katheter herum an dem Katheter angeordnet sind und die in Längsrichtung (longitudinaler Richtung) an den Penisschaft und optional an die Eichel (*Glans penis*) anlegbar sind. Der Katheter ist insbesondere für die intermittierende Selbst-Katheterisierung bei einem männlichen, menschlichen Individuum geeignet.

Wie zuvor beschrieben, sind Katheter zur intermittierenden (Selbst-)Katheterisierung für männliche, menschliche Individuen bekannt. Gegenüber diesen bekannten ISK Kathetern bringen die erfindungsgemäßen Katheter jedoch den Vorteil mit sich, dass sie durch die flexiblen, länglichen Halteelemente leicht am Penis fixiert und auch von motorisch eingeschränkten Patienten mit nur einer Hand gehalten werden können. Gleichzeitig kann durch das Halten und Fixieren der Halteelemente am Penis der Penisschaft und die Harnröhre gestreckt und damit begradigt werden, so dass das Einführen des Kathetertubus in die Harnröhre vereinfacht und das Verletzungs- und Infektionsrisiko minimiert wird.

Der Begriff intermittierende (Selbst-)Katheterisierung bezeichnet die intermittierende (Selbst-)Ableitung von Urin mittels eines Katheters. Ein Katheter im Sinne der Erfindung ist somit ein Harnblasenkatheter, d. h. ein Katheter zur Ableitung von Urin. Als intermittierende Selbst-Katheterisierung wird insbesondere die vom Patienten selber durchgeführte Eigenkatheterisierung bezeichnet, die regelmäßig wiederholt wird. Die erfindungsgemäßen Katheter können selbstverständlich auch für die Katheterisierung am Patienten durch medizinisches Fachpersonal eingesetzt werden. Die Verwendung für die Selbst-Katheterisierung ist bevorzugt. Die zeitlichen Abstände der Wiederholung (die Frequenz) sind individuell und je nach Beschwerden, der Trinkmenge und den vom Patienten eingenommenen Medikamenten anzupassen. In der Regel wird die Katheterisierung vier bis sechs Mal am Tag durchgeführt.

Auf Grund der anatomischen Unterschiede zwischen den Urogenitalsystemen von Mann und Frau sind Katheter in der Regel nur für Personen eines Geschlechts geeignet. Die erfindungsgemäßen Katheter sind speziell an das Urogenitalsystem des Mannes angepasst und somit für die Anwendung durch einen männlichen ISK Patienten bzw. ein männliches, menschliches Individuum geeignet.

Der erfindungsgemäße Katheter umfasst einen Kathetertubus. Der Kathetertubus ist röhren- bzw. schlauch-artig ausgebildet. Durch den Kathetertubus hindurch kann Urin abfließen bzw. abgeleitet werden. Geeignete Kathetertuben sind dem Fachmann bekannt. Die Kathetertuben weisen eine Länge auf, die das Erreichen der Harnblase und insbesondere das Überwinden des Blasenschließmuskels ermöglicht. Bevorzugt weist der Kathetertubus daher eine Länge von 25 cm oder mehr, vorzugsweise von 30 cm oder mehr, stärker bevorzugt von 35 cm oder mehr, z. B. von etwa 41 cm, auf. Anders gesagt weist der Kathetertubus eine Länge von 25 cm bis 60 cm, bevorzugt von 30 cm bis 50 cm auf. Für männliche Kinder oder Jugendliche können diese Längen selbstverständlich entsprechend angepasst werden. Kathetertuben für Kinder können z. B. eine Länge von 15 cm oder mehr, bevorzugt von 18 cm oder mehr, wie etwa 22 cm aufweisen. Anders gesagt weisen Kathetertuben für Kinder z. B. eine Länge von 15 cm bis 29 cm, bevorzugt von 18 cm bis 26 cm auf.

Der Kathetertubus weist ferner eine Steifigkeit auf, die sicherstellt, dass der Kathetertubus nicht durch den Blasenschließmuskel zusammengedrückt und damit verschlossen wird. Entsprechende Kathetertuben sind dem Fachmann bekannt.

Der Außendurchmesser des Kathetertubus kann 6 bis 20 Charriere, bevorzugt 14 bis 18 Charriere betragen. Drei Charriere entsprechen 1 mm. Kathetertuben für männliche Kinder weisen bevorzugt einen Außendurchmesser von 6 bis 10 Charriere auf.

Geeignete Materialien und (z. B. hydrophile) Beschichtungen für Kathetertuben sind dem Fachmann bekannt. Erfindungsgemäß kann jegliches in diesem Zusammenhang geeignete Material verwendet werden.

Der Katheter umfasst ferner ein Einführelement. Geeignete Einführelemente sind dem Fachmann ebenfalls bekannt und werden in jüngerer Zeit bereits für Katheter eingesetzt.

Das Einführelement weist an seinem distalen Ende einen konusförmigen Teil mit einer abgerundeten Spitze auf (die Vorlaufspitze). Die Vorlaufspitze weist eine verschließbare Öffnung (eine Tubusdurchführung) auf, die das Durchschieben des Kathetertubus ermöglicht und gleichzeitig den Eintrag von Keimen im verschlossenen Zustand minimiert. Die Vorlaufspitze kann in die Harnröhre eingeführt werden und weist einem diesem Zweck entsprechenden Durchmesser und Länge auf. Die Tubusdurchführung kann z. B. aus einem oder mehreren, einander kreuzenden Schlitzen bestehen, d.h. z. B. kreuzförmig oder sternförmig ausgebildet sein.

Proximal schließt sich an den konusförmigen Teil ein diesen Teil kreisförmig umlaufender Kragen an. Der Kragen weist einen Außendurchmesser auf, der groß genug ist, um das Einführen des Einführelements zu stoppen, d. h. der größer ist (z. B. doppelt so groß) als der Durchmesser der Harnröhre.

Weiter proximal schließt sich an den Kragen ein länglicher Spitzenhalter an, der mindestens abschnittsweise röhrenartig ausgebildet ist und den Kathetertubus umgibt. Der Spitzenhalter dient der Stabilisierung des Tubusteils und des Einführelements sowie der leichteren Handhabbarkeit durch den Anwender. Der Spitzenhalter kann z. B. eine Trichterform aufweisen, wobei die breitere Trichterseite sich proximal an den Kragen anschließt.

Das Einführelement kann einstückig ausgebildet sein oder aus verschiedenen Elementen zusammengesetzt, z. B. geklebt, geklemmt oder gesteckt sein. So können z. B. Spitzenhalter, Kragen und/oder Vorlaufspitze jeweils einzelne Teile sein. Entsprechend ist angedacht, die unterschiedlichen Teile eines Einführelements aus unterschiedlichen Materialien auszubilden. So kann die Vorlaufspitze z. B. aus einem anderen Material hergestellt sein, als der Spitzenhalter und der Kragen. Derartige Ausführungen sind dem Fachmann bekannt.

Das Einführelement bzw. der Spitzenhalter, der Kragen und/oder die Vorlaufspitze bestehen vorzugsweise aus einem Kunststoff, wie z. B. Silikon. Vorzugsweise besteht das Einführelement aus Spitzenhalter, Kragen und Vorlaufspitze.

Im unbenutzten Zustand ist das distale Ende des Kathetertubus' in die Vorlaufspitze des Einführelements eingeführt. Die Vorlaufspitze kann dann in die Harnröhre eingeführt werden. Anschließend wird das distale Ende des Kathetertubus' durch das Einführelement, und insbesondere die Vorlaufspitze, sowie durch die Harnröhre (Urethra) bis in die Harnblase eingeführt. Das Einführen und Anlegen des erfindungsgemäßen Katheters geschieht bevorzugt nach Zurückziehen der Vorhaut, um eine stabile Halterung des Katheters zu gewährleisten.

Der Katheter umfasst in seinem distalen Endbereich zwei oder mehr flexible, längliche Halteelemente, deren proximale Enden um den Katheter herum am Katheter angeordnet sind und die in Längsrichtung (longitudinaler Richtung) an den Penisschaft und optional an die Eichel (*Glans penis*) anlegbar sind. Die Halteelemente sind somit derart ausgebildet, dass sie im Wesentlichen parallel zur longitudinalen Achse (Längsachse) des Penis an den Penisschaft angelegt werden können.

Der distale Endbereich des Katheters bezeichnet den Bereich des Katheters, der sich nach dem Einführen der Vorlaufspitze in dem Penis und in unmittelbarer Nähe der Penisspitze befindet, z. B. nach dem Einführen der Vorlaufspitze 3 cm oder weniger, bevorzugt 2 cm oder weniger, stärker bevorzugt 1 cm oder weniger von der Penisspitze entfernt ist. Sind die Halteelemente in Richtung des Penis geklappt oder an den Penisschaft angelegt, so sind die Halteelemente selber Teil des distalen Endbereichs des Katheters. Mit anderen Worten ist der distale Endbereich des Katheters derjenige, der bei Anwendung durch medizinisches Fachpersonal in der Regel am Weitesten von dem medizinischen Fachpersonal entfernt ist. Dies entspricht der Standardbenennung auf dem Fachgebiet der Katheter.

Die Halteelemente weisen eine längliche Form mit einem distalen und einem proximalen Ende auf. Als distal wird dasjenige Ende des Katheters bzw. der Halteelemente bezeichnet, das während der Katheterisierung mit dem Katheter in der Regel am weitesten vom Körper des medizinischen Fachpersonal bzw. am wenigsten weit vom Körper des männlichen Patienten entfernt sein wird. Als distal wird dasjenige Ende des Katheters bezeichnet, das während der Ableitung von Urin in den Penis eingeführt wird.

Als das proximale Ende eines Halteelements wird dasjenige Ende bezeichnet, das während der Selbstableitung von Urin am wenigsten weit von der Körpermitte des medizinischen Fachpersonals entfernt ist, d. h. dasjenige Ende, das am Katheter befestigt ist. Das distale Ende eines Halteelements ist entsprechend dasjenige, das mit an den Penisschaft angelegt und gehalten wird.

Als distal wird dasjenige Ende des Penisschafts des Patienten bezeichnet, das am weitesten von der Körpermitte des Patienten entfernt ist.

Die Halteelemente sind mit ihrem proximalen Ende im distalen Endbereich des Katheters angeordnet bzw. angebracht. Vorzugsweise sind die Halteelemente an einem relativ zum Einführelement nicht oder nur wenig beweglichen Teil des Katheters angebracht. So sind die Halteelemente z. B. nicht an dem Kathetertubus befestigt, da dieser in die Harnröhre eingeschoben werden soll. Bevorzugt sind die proximalen Enden der Halteelemente an dem Einführelement, insbesondere an dem Kragen oder Spitzenhalter, angebracht. Dabei ist es besonders bevorzugt, dass die Halteelemente mit ihrem proximalen Ende an dem Kragen des Einführelements oder angrenzend an den Kragen des Einführelements, z. B. an dem Spitzenhalter, befestigt sind. Alternativ können die Halteelemente allerdings auch z. B. mit einer Umhüllung fest verbunden sein, welche den Kathetertubus umgibt und z. B. ein Gleitgel enthält.

Halteelemente und Einführelement bzw. Halteelemente und Kragen des Einführelements können einteilig ausgeprägt sein. Alternativ können die Halteelemente mechanisch mittels eines Befestigungsmittels an dem Einführelement befestigt sein, z. B. distal oder proximal angrenzend an dem Kragen des Einführelements. Halteelement und Einführelement grenzen dabei aneinander an. Dies schließt selbstverständlich nicht aus, dass Halteelement und Einführelement durch ein dünnes Befestigungsmittel, wie z. B. Klebstoff, voneinander getrennt sein können.

Die mechanische Befestigung der Halteelemente an dem Einführelement kann entsprechend z. B. durch klemmen oder kleben ausgeführt sein. So kann z. B. proximal der Halteelemente ein weiteres, das Einführelement ringförmig angrenzend umgebendes Klemm- oder Klebelement an dem Einführelement angebracht sein. Das Klemm- oder Klebelement kann z. B. ringförmig ausgebildet sein. Die Befestigung des Klemmelements am dem Einführelement kann dadurch sichergestellt sein, dass das Klemmelement sehr eng an der Außenfläche des Einführelements anliegt. Das Klebelement kann durch einen üblichen Klebstoff mit dem Einführelement und/oder den Halteelementen verklebt sein. Bevorzugt grenzt das Befestigungsmittel, z. B. das Klemm- oder Klebelement, unmittelbar proximal an das proximale Ende der Halteelemente an.

Die Halteelemente sind flexibel und weisen eine längliche Form auf. Insbesondere sind die Halteelemente derart flexibel, dass sie sich flach aneinander gelegt verpacken lassen (und an den Penisschaft anlegen lassen). Beim Anlegen an den Penisschaft passen sich die Halteelemente sowohl der Form des Penis und seinen Bewegungen, als auch den Bewegungen der haltenden Hand an. Gleichzeitig sind die Halteelemente so stabil, dass sie bei normalem Gebrauch nicht reißen oder anderweitig zerstört werden. Die Flexibilität der Halteelemente stellt sicher, dass der Penis sicher durch die haltende Hand stabilisiert werden kann und gleichzeitig der Katheter nicht verrutscht.

Geeignete Materialien, aus denen die Halteelemente hergestellt werden können, kann der Fachmann anhand dieser Erfordernisse bestimmen. Es ist besonders bevorzugt, dass die Halteelemente aus einem flexiblen Kunststoff sind. Der flexible Kunststoff wird in der Regel biokompatibel sein. Vorzugsweise ist der Kunststoff ein elastomerer Kunststoff. Weiter bevorzugt ist es, dass die Halteelemente aus einem biegeweichen Kunststoff ausgebildet sind. Auf diese Weise können die Halteelemente mit ihrer gesamten Innenfläche an Penisse unterschiedlicher Größen angelegt werden. Die "Innenfläche" des Halteelements ist die Fläche, die bei Anlegung eines Halteelements an den Penis der Längsachse des Penisschafts bzw. deren proximaler Verlängerung zugewandt ist. Die Innenfläche beginnt vorzugsweise unmittelbar an der Stelle, an der das Halteelement mit dem Einführelement verbunden ist. Geeignete flexible Kunststoffe können z. B. ausgewählt sein aus der Gruppe bestehend aus Polyurethanen (PU), thermoplastischen Elastomeren (TPE) und Silikonen.

In einer bevorzugten Ausführungsform umfasst die Oberfläche der Halteelemente ein rutschfestes Material. Die Halteelemente können zur Gänze aus diesem rutschfesten Material bestehen oder nur mit diesem beschichtet sind. Alternativ kann die Oberfläche der Halteelemente auch so strukturiert sein, dass ein Verrutschen verhindert wird, z. B. durch Noppung der Oberfläche.

Um die einfache Handhabung des Katheters auch durch motorisch eingeschränkte Patienten zu gewährleisten, ist es jedoch nicht erwünscht, dass die Halteelemente an dem Penis kleben. Die Halteelemente weisen somit bevorzugt keine selbstklebende Oberfläche auf.

Die Halteelemente können eine Dicke von etwa 0,05 bis 3 mm aufweisen, vorzugsweise 0,1 bis 1 mm, wie z. B. etwa 0,5 mm. Die Dicke kann je nach verwendetem Material angepasst werden.

"Länglich" bedeutet, dass die Halteelemente eine im Verhältnis zur Breite größere Länge aufweisen. Hierdurch wird sichergestellt, dass die Halteelemente auf einer ausreichenden Länge an den Penisschaft angelegt werden können. Als proximales bzw. distales Ende des Halteelements werden die sich gegenüberliegenden schmaleren Enden des länglichen Halteelements bezeichnet. Beim Anlegen der Halteelemente an den Penisschaft werden die Längsachsen der Halteelemente in etwa parallel zur Längsachse des Penis ausgerichtet.

Die Halteelemente können recht- oder vieleckig sein, weisen jedoch vorzugsweise - mindestens am distalen Ende - abgerundete Ecken auf. Weiter vorzugsweise weisen die Halteelemente eine kronblattartige Form auf, z. B. die Form von Kronblättern von *Tulipa kaufmanniana* (Seerosen-Tulpe).

Nachdem das Einführelement in die Harnröhre eingeführt wurde und die Halteelemente an den Penisschaft angelegt wurden, weisen die Halteelemente vorzugsweise gemeinsam eine unterbrochene Blütenkelchform auf. Die proximalen Enden der Halteelemente können z. B. derart um den Katheter herum angeordnet sein, dass zwischen der Mitte des proximalen Endes eines ersten Halteelements und der Mitte des proximalen Endes eines zweiten Halteelements ein Winkel von 90° bis 180° liegt, vorzugsweise von 120° bis 180°.

Der Katheter kann z. B. (genau) zwei Halteelemente aufweisen. In einer Ausführungsform weist der Katheter zwei Haltelemente auf, die derart um den Katheter herum am Katheter angeordnet sind, dass zwischen der Mitte des proximalen Endes des ersten Halteelements und der Mitte des proximalen Endes des zweiten Halteelements ein Winkel von 90° bis 180° liegt, vorzugsweise von 120° bis 180°, besonders bevorzugt etwa 180°C.

Dabei weisen die Halteelemente eine Breite auf, die ausreichend ist, um eine Stabilisierung des Katheters an dem Penis zu gewährleisten und gleichzeitig sicherzustellen, dass beim Einführen der Vorlaufspitze in die Harnröhre der Blick auf die Einführstelle, d. h. die Harnröhrenöffnung, frei bleibt. Um letzterem Erfordernis Rechnung zu tragen, sind die Halteelemente bevorzugt derart ausgebildet, dass sie beim Anlegen um den Penis von 90° bis 270° des Penisumfangs am Eichelrand (*Corona glandis*) bedecken, besonders bevorzugt von 90°C bis 180° des Penisumfangs.

Die Halteelemente können, müssen sich aber nicht, im an den Penisschaft angelegten Zustand überlappen. Die Halteelemente können an ihrer breitesten Stelle eine Breite von z. B. 1,5 bis 4 cm aufweisen, vorzugsweise zwischen 2 und 3 cm, wie etwa 2,5 cm. Die Halteelemente können an ihrer längsten Stelle eine Länge von z. B. 4 cm oder mehr, bevorzugt 6 cm oder mehr aufweisen, z. B. 6,5 cm. Mit anderen Worten können die Halteelemente an der genannten Stelle eine Länge von 4 bis 10 cm, vorzugsweise von 6 bis 8 cm aufweisen. Diese Länge stellt eine sichere Fixierung durch die Hand des Patienten an dem Penisschaft sicher.

Die einzelnen Halteelemente können separate Teile sein. Alternativ können jedoch auch zwei oder mehr Halteelemente, bevorzugt alle Halteelemente eines erfindungsgemäßen Katheters gemeinsam aus einem Stück gefertigt sein. Anders gesagt, wären in dieser Ausführungsform alle (oder zwei oder mehr) in einem erfindungsgemäßen Katheter enthaltenen Halteelemente gemeinsam als ein Teil ausgebildet. Die zwei oder mehr Haltelemente können z. B. als ein Teil aus einem Material gegossen, gedruckt oder ausgestochen werden. Anschließend oder gleichzeitig kann ein Loch mit dem Durchmesser des Spitzenhalters zwischen den Halteelementen eingefügt werden, durch das der Spitzenhalter hindurchgeschoben und befestigt (z. B. geklemmt oder geklebt) werden kann.

Der Katheter kann ferner eine Umhüllung für den Kathetertubus umfassen, wobei die Umhüllung ein Gleitmittel enthält. Geeignete Materialien für die Umhüllung und geeignete Gleitmittel sind dem Fachmann bekannt.

Der Katheter kann darüber hinaus in eine sterile Umverpackung verpackt sein. So wird eine Keimbelastung des Katheters vor dem Auspacken durch den Benutzer im Wesentlichen ausgeschlossen und das Infektionsrisiko deutlich reduziert. Geeignete sterile Umverpackungen sind dem Fachmann bekannt.

In einem weiteren Aspekt betrifft die Erfindung ein Verfahren zur intermittierenden Selbst-Katheterisierung, dadurch gekennzeichnet, dass das Verfahren Schritte umfasst, bei denen man: a) die Vorlaufspitze des Einführelements eines Katheters nach einem der vorangegangenen Ansprüche in die Harnröhre einführt; b) die Halteelemente in Längsrichtung an den Penisschaft und optional an die Eichel (*Glans penis*) anlegt; c) die Halteelemente und den Penis mit einer Hand, vorzugsweise zwischen dem Daumen und einem oder mehreren zu dem Daumen in Oppositionsstellung stehenden Fingern, festhält; d) optional den Penisschaft streckt; und e) den Kathetertubus in die Harnröhre einführt. "Longitudinale" oder Längs-Richtung bezeichnet die Richtung, die parallel zur Längsachse des Penis verläuft.

Schritt b) kann vor, nach oder gleichzeitig mit Schritt a) durchgeführt werden. In Schritt b) werden die Halteelemente zwischen zwei oder mehr Fingern oder in der Handfläche gehalten. Ebenso kann Schritt c) während oder nach Schritt a) durchgeführt werden.

In ähnlicher Weise findet Schritt c) vorzugsweise mindestens während des Schritts e) (und/oder d)) statt bzw. wird während der Durchführung von Schritt d) und/oder e) fortgesetzt. Darüber hinaus wird Schritt c) bevorzugt während der an Schritt e) anschließenden Ableitung von Urin durch den Kathetertubus fortgesetzt. Auf welche Weise die Halteelemente durch den Patienten am Penisschaft festgehalten werden, kann je nach den motorischen Einschränkungen des Patienten variiert werden.

In Schritt d) kann der Penisschaft gestreckt werden. Dies geschieht durch einfaches Festhalten und Streckung in der Handfläche des Anwenders. Schritt d) kann vor oder während Schritt e) durchgeführt werden, d. h. die Streckung des Penis' kann vor oder während der Einführung des Kathetertubus durchgeführt werden.

### Kurze Beschreibung der Figuren

In den Zeichnungen sind Ausführungsbeispiele der Erfindung schematisch dargestellt. Es zeigen:
- **Fig. 1**: einen erfindungsgemäßen Katheter und einen Penis, wobei die Vorlaufspitze und die Harnröhrenöffnung aufeinander ausgerichtet sind; und
- **Fig. 2**: den erfindungsgemäßen Katheter aus Fig. 1 in Aufsicht auf die Tubusdurchführung der Vorlaufspitze.

### Ausführungsbeispiele

Weitere Vorteile, Kennzeichen und Merkmale der vorliegenden Erfindung werden bei der nachfolgenden detaillierten Beschreibung von Ausführungsbeispielen anhand der beigefügten Zeichnungen deutlich. Allerdings ist die Erfindung nicht auf diese Ausführungsbeispiele beschränkt.

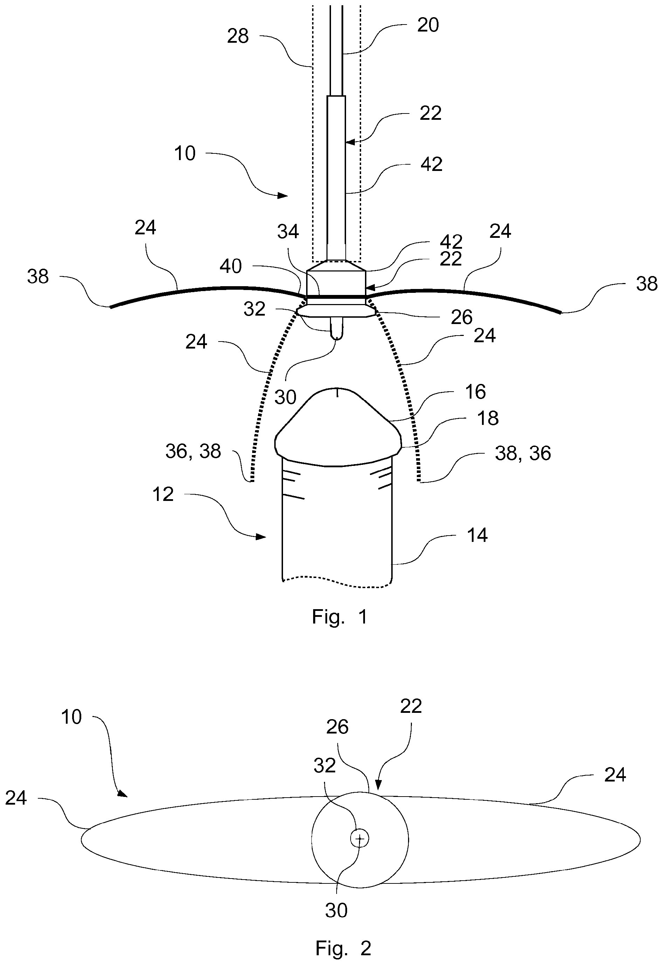

Fig. 1 zeigt ein Verfahren zur Anwendung eines erfindungsgemäßen Katheters 10. Es ist erkennbar, dass die Vorlaufspitze 32 des Katheters 10 auf die Harnröhrenöffnung des Penis 12 ausgerichtet ist, so dass die Vorlaufspitze 32 in die Harnröhre eingeführt werden kann. Aufgrund der besseren Darstellbarkeit und insbesondere Sichtbarkeit der einzelnen Elemente ist keine menschliche Hand dargestellt, die den Penis 12 und die Halteelemente 24 zur Einführung der Vorlaufspitze 32 hält.

Der Katheter 10 weist einen Kathetertubus 20 auf, der durch ein Einführelement 22 verläuft. Innerhalb des Einführelements 22 verläuft der Kathetertubus 20 bis kurz vor der Tubusdurchführung 30 und kann nach Einführung der Vorlaufspitze 32 in die Harnröhre einfach durch die Tubusdurchführung 30 hindurchgeschoben werden.

Der Kathetertubus 20 ist in eine Umhüllung 28 eingeschweißt, die ein Gleitgel enthält. Die Umhüllung 28 kann an ihrem distalen Ende mit einer oder mehreren Schweißnahten verschlossen sein. Ebenso kann die Umhüllung auch an ihrem proximalen Ende (nicht dargestellt) durch eine Schweißnaht verschlossen sein. An seinem proximalen Ende (nicht dargestellt) weist der Katheter in der Regel einen Trichter oder ein anderes Element auf, das bei der Ableitung des Urins in einen anderen Behälter oder in eine Entsorgungseinrichtung behilflich sein kann.

Das Einführelement 22 weist einen Spitzenhalter 42 und weiter distal einen Kragen 26 auf, der umlaufend im distalen Endbereich des Einführelements 22 ausgebildet ist. Weiter distal weist das Einführelement 22 eine Vorlaufspitze 32 auf, die zur Einführung in die Harnröhre geeignet ist. Die Vorlaufspitze 32 weist an ihrem distalen Ende eine abgerundete Form auf, um das Verletzungsrisiko zu reduzieren. Darüber hinaus ist das distale Ende der Vorlaufspitze 32 mit einer Tubusdurchführung 30 versehen, durch die der Kathetertubus 20 hindurchgeführt und in die Harnröhre und die Harnblase vorgeschoben werden kann.

Proximal des Kragens 26 sind zwei Haltelemente 24 an dem Einführelement 22 bzw. dem Spitzenhalter 42 angebracht. In dem gezeigten Ausführungsbeispiel sind die Halteelemente 24 durch einen um das Einführelement umlaufenden Ring, der z. B. aus demselben Material sein kann wie die Halteelemente 24, an dem Einführelement 22 befestigt. Alternativ können die Halteelemente einteilig mit dem Einführelement 22 ausgebildet sein. Die beiden Halteelemente 24 sind in Fig. 1 im abgeklappten Zustand (durchgezogene, dicke Linie) dargestellt, in dem die Halteelemente 24 zu Penis und Kathetertubus 20 im Wesentlichen orthogonal verlaufen. Da die Halteelemente 24 sehr flexibel sind, können sie flexibel in eine Position bewegt werden, in der die Längsachse der Halteelemente 24 im Wesentlichen parallel zur Längsrichtung des Penis ausgerichtet ist und die Halteelemente an den Penisschaft 14 angelegt sind. In einer gestrichelten, dicken Linie sind die beiden Halteelemente 24 gezeigt, die gerade in eine solche an den Penisschaft angelegte Position bewegt werden. Die Halteelemente 24 (gestrichelte, dicke Linie) sind bereits im Wesentlichen parallel zu der Längsachse des Penisschaft 14 ausgerichtet, aber noch nicht an den Penisschaft 14 angelegt.

Es ist erkennbar, dass die Halteelemente 24 eine Länge aufweisen, die es erlaubt, die Halteelemente 24 in longitudinaler Richtung an die Eichel 16 und insbesondere entlang des Penisschafts 14 anzulegen und mit einer Hand festzuhalten. Es ist erkennbar, dass der Blick auf die einzuführende Vorlaufspitze 32 in dieser Konfiguration frei sein wird.

Fig. 2 zeigt den erfindungsgemäßen Katheter 10 aus Fig. 1 in Aufsicht auf die Tubusdurchführung 30 der Vorlaufspitze 32. Der dargestellte Katheter 10 weist ein Einführelement 22 auf, das einen Kragen 26 und eine Vorlaufspitze 32 umfasst, die in distaler Richtung (d. h. in Richtung des Körpers des Individuums) von dem Kragen 26 ausgebildet ist. Die Vorlaufspitze 32 weist eine kreuzförmige Tubusdurchführung 30 auf. Um ein einfaches und reversibles Durchschieben der Tubusdurchführung 30 zu ermöglichen, ist die Vorlaufspitze 32 aus einem flexiblen Kunststoff gefertigt. Die Spitze der Vorlaufspitze 32 wird sich flexibel öffnen, wenn ein Druck aus dem Inneren des Einführelements 22 durch das distale Ende des Kathetertubus 20 auf die Spitze ausgeübt wird. Die distale Spitze der Vorlaufspitze 32 wird sich wieder schließen, sobald der Kathetertubus 20 aus der Tubusdurchführung 30 herausgeschoben bzw. in proximaler Richtung zurückgezogen ist.

Der Katheter 10 in Fig. 2 weist zwei Halteelemente 24 auf. Die Halteelemente 24 befinden sich in etwa in der Position, die in Fig. 1 mit den dicken, durchgezogenen Linien dargestellt ist, d.h. im Wesentlichen orthogonal zur Längsrichtung von Penis 12 und Kathetertubus 20. Die beiden Halteelemente 24 sind derart um den Katheter herum angeordnet, dass zwischen der Mitte des proximalen Endes eines ersten Halteelements 24 und der Mitte des proximalen Endes eines zweiten Halteelements 24 ein Winkel von etwa 180° liegt. Die proximalen Enden der Halteelemente 24 sind die Enden, die an dem Einführelement angebracht sind. Die Halteelemente weisen eine längliche und einfache kronblattartige Form auf, d. h. die Form eines einfachen Kronblatts, z. B. die eines Gänseblümchenkronblatts.

Obwohl die vorliegende Erfindung anhand der Ausführungsbeispiele detailliert beschrieben worden ist, ist für den Fachmann selbstverständlich, dass die Erfindung nicht auf diese Ausführungsbeispiele beschränkt ist, sondern dass vielmehr Abwandlungen in der Weise möglich sind, dass einzelne Merkmale weggelassen oder andersartige Kombinationen der vorgestellten Einzelmerkmale verwirklicht werden können, sofern der Schutzbereich der beigefügten Ansprüche nicht verlassen wird. Die vorliegende Offenbarung schließt sämtliche Kombinationen der vorgestellten Einzelmerkmale ein.

**Bezugszeichenliste**

| | | | |
|---|---|---|---|
| 10 | Katheter | 36 | distales Ende Katheter |
| 12 | Penis | 38 | distales Ende Halteelement |
| 14 | Penisschaft | 40 | proximales Ende Halteelement |
| 16 | Eichel (*Glans penis*) | 42 | Spitzenhalter |
| 18 | Eichelrand (*Corona glandis*) | | |
| 20 | Kathetertubus | | |
| 22 | Einführelement | | |
| 24 | Halteelement | | |
| 26 | Kragen | | |
| 28 | Umhüllung | | |
| 30 | Tubusdurchführung | | |
| 32 | Vorlaufspitze | | |
| 34 | Befestigungsring | | |

## Patentansprüche

1. Katheter (10) zur intermittierenden Katheterisierung, insbesondere zur intermittierenden Selbst-Katheterisierung, wobei der Katheter (10) einen Kathetertubus (20) und ein Einführelement (22) umfasst, **dadurch gekennzeichnet, dass** der Katheter (10) in seinem distalen Endbereich zwei oder mehr flexible, längliche Halteelemente (24) umfasst, deren proximale Enden (40) um den Katheter (10) herum an dem Katheter (10) angeordnet sind und die in Längsrichtung an den Penisschaft (14) und optional an die Eichel (*Glans penis*) (16) anlegbar sind.

2. Katheter (10) nach Anspruch 1, dessen Einführelement (22) einen Kragen (26) aufweist, **dadurch gekennzeichnet, dass** die proximalen Enden (40) der Halteelemente (24) an dem Einführelement (22) angebracht sind, bevorzugt an dem Kragen (26) des Einführelements (22) oder angrenzend an den Kragen (26) des Einführelements (22).

3. Katheter (10) nach einem der Ansprüche 1 oder 2, **dadurch gekennzeichnet, dass** die proximalen Enden (40) der Halteelemente (24) derart um den Katheter (10) herum angeordnet sind, dass zwischen der Mitte des proximalen Endes (40) eines ersten Halteelements (24) und der Mitte des proximalen Endes (40) eines zweiten Halteelements (24) ein Winkel von 90° bis 180° liegt, vorzugsweise von 120° bis 180°.

4. Katheter (10) nach einem der vorangegangenen Ansprüche, **dadurch gekennzeichnet, dass** die Halteelemente (24) aus einem flexiblen Kunststoff bestehen.

5. Katheter (10) nach einem der vorangegangenen Ansprüche, **dadurch gekennzeichnet, dass** der Katheter (10) zwei Halteelemente (24) aufweist.

6. Katheter (10) nach einem der vorangegangenen Ansprüche, **dadurch gekennzeichnet, dass** die Halteelemente (24) derart ausgebildet sind, dass sie beim Anlegen um den Penis (12) 90° bis 270° des Penisumfangs am Eichelrand (18) (*Corona glandis*) bedecken.

7. Katheter (10) nach einem der vorangegangenen Ansprüche, **dadurch gekennzeichnet, dass** die Halteelemente (24) eine Länge von 6 cm oder mehr aufweisen.

8. Katheter (10) nach einem der vorangegangenen Ansprüche, **dadurch gekennzeichnet, dass** die Halteelemente (24) eine kronblattartige Form aufweisen, bevorzugt die Form von Kronblättern von *Tulipa kaufmanniana.*

9. Katheter (10) nach einem der vorangegangenen Ansprüche, **dadurch gekennzeichnet, dass** der Katheter (10) eine Umhüllung (28) für den Kathetertubus (20) umfasst, wobei die Umhüllung (28) ein Gleitmittel enthält, und wobei der Katheter (10) vorzugsweise in eine sterile Umverpackung verpackt ist.

10. Verfahren zur intermittierenden Katheterisierung, insbesondere zur intermittierenden Selbst-Katheterisierung, **dadurch gekennzeichnet, dass** das Verfahren Schritte umfasst, bei denen man:
a) die Vorlaufspitze (32) des Einführelements (22) eines Katheters (10) nach einem der vorangegangenen Ansprüche in die Harnröhre einführt;
b) die Halteelemente (24) in Längsrichtung an den Penisschaft (14) und optional an die Eichel (16) (*Glans penis*) anlegt;
c) die Halteelemente (24) und den Penis (12) mit einer Hand, vorzugsweise zwischen dem Daumen und einem oder mehreren zu dem Daumen in Oppositionsstellung stehenden Fingern, festhält;
d) optional den Penisschaft (14) streckt; und
e) den Kathetertubus (20) in die Harnröhre einführt.
